(19) Europäisches Patentamt
European Patent Office
Office européen des brevets

(11) Publication number: **0 060 012**
**B1**

(12) **EUROPEAN PATENT SPECIFICATION**

(45) Date of publication of patent specification: **29.08.84**

(51) Int. Cl.$^3$: **C 07 D 471/04** // A61K31/44 ,(C07D471/04, 221/00, 221/00)

(21) Application number: **82200534.4**

(22) Date of filing: **10.04.80**

(60) Publication number of the earlier application in accordance with Art. 76 EPC: **0 018 735**

(54) **4-Halo-1,8-naphthyridine derivatives useful as intermediates and processes for their preparation.**

(30) Priority: **18.04.79 US 31255**
**28.02.80 US 125600**

(43) Date of publication of application:
**15.09.82 Bulletin 82/37**

(45) Publication of the grant of the patent:
**29.08.84 Bulletin 84/35**

(84) Designated Contracting States:
**AT BE CH DE FR IT LI LU NL SE**

(56) References cited:
**None**

(73) Proprietor: **AMERICAN HOME PRODUCTS CORPORATION**
**685, Third Avenue**
**New York, New York 10017 (US)**

(72) Inventor: **Santilli, Arthur Attilio**
**1737 Sue Ellen Drive**
**Havertown Pennsylvania (US)**
Inventor: **Scotese, Anthony Carmen**
**240 Beidler Road**
**King of Prussia Pennsylvania (US)**

(74) Representative: **Wileman, David Francis et al**
**c/o John Wyeth and Brother Limited**
**Huntercombe Lane South**
**Taplow Maidenhead Berkshire SL6 OPH (GB)**

**EP 0 060 012 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to 1,8-naphthyridine derivatives.

More particularly this invention relates to a group of 4-halo-3-substituted-1,2-dihydro-2-oxo-1, 8-naphthyridine derivatives which are useful as intermediates to a group of compounds which act as gastric anti-secretory agents and may be used in the treatment of peptic ulcer disease.

In our European Patent Application No. 80301157.6 (Publication No. 18735) there are described and claimed 4-amino and 4-substituted amino-1,8-naphthyridine derivative of formula

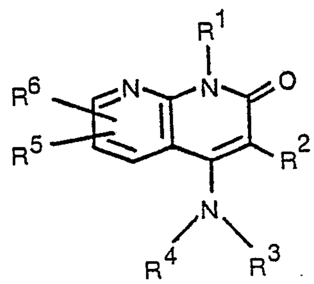

(I)

in which $R^1$ is hydrogen, alkyl of 1 to 6 carbon atoms, cycloalkyl of 5 to 6 carbon atoms, alken-(3,4,5 or 6)-yl of 3 to 6 carbon atoms or alkyn-(3,4,5 or 6)-yl of 3 to 6 carbon atoms; $R^2$ is —CN, —$CO_2H$, —$CO_2R^7$ where $R^7$ is alkyl of 1 to 6 carbon atoms, —$CONHNH_2$ or

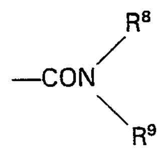

where $R^8$ and $R^9$ are independently hydrogen or alkyl of 1 to 6 carbon atoms; $R^3$ and $R^4$ are, independently, hydrogen, alkyl of 1 to 6 carbon atoms, hydroxyalkyl of 1 to 6 carbon atoms, dialkylaminoalkyl of 4 to 8 carbon atoms, alkanoyl of 2 to 4 carbon atoms, haloalkanoyl of 2 to 4 carbon atoms, or when taken together with the nitrogen atom to which they are attached, $R^3$ and $R^4$ complete a heterocyclic group selected from aziridinyl, azetidinyl, pyrrolidinyl, piperidinyl, piperazinyl, 4-lower alkylpiperazinyl, 4-phenylpiperazinyl, 4-[1-ethyl-1,2-dihydro-7-methyl-2-oxo-1,8-naphthyridine-4-yl-3-carboxylic acid ethyl ester]piperazinyl, 4-carb(lower) alkoxypiperazinyl, morpholinyl, thiomorpholinyl or a ring substituted lower alkyl analogue thereof; $R^5$ and $R^6$ are independently hydrogen, alkyl of 1 to 4 carbon atoms, halo, alkylamino of 1 to 4 carbon atoms, or alkoxy of 1 to 6 carbon atoms; and pharmaceutically acceptable salts thereof.

When either $R^1$ or $R^2$ is alkyl, they preferably have 1 to 4 carbon atoms. Preferably $R^3$ and $R^4$ are both hydrogen or together with the nitrogen atom represent pyrrolidinyl, morpholinyl, piperazinyl or 4-methylpiperazinyl. Preferably $R^5$ is hydrogen and $R^6$ is hydrogen or methyl.

The preferred compounds of formula I from the standpoint of potency as anti-secretory agents are those of the formula:

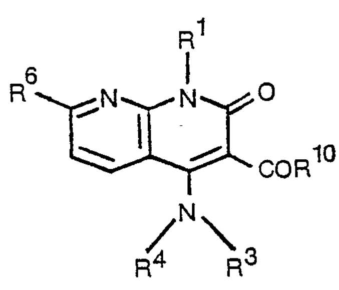

(II)

in which $R^1$ is hydrogen, methyl, ethyl, propyl, allyl, propargyl, cyclohexyl or isobutyl; $R^3$ is hydrogen, alkyl of 1 to 6 carbon atoms or trifluoroacetyl; $R^4$ is hydrogen, alkyl of 1 to 6 carbon atoms, or when taken with $R^3$ and the nitrogen atom to which they are attached forms a heterocyclic group selected from pyrrolidinyl, morpholinyl, piperazinyl, or 4-methyl-piperazinyl; $R^6$ is hydrogen or methyl; and $R^{10}$ is hydroxy, alkoxy of 1 to 6 carbon atoms or hydrazinyl; or a pharmaceutically acceptable salt thereof.

The term halo means chloro, bromo, iodo or fluoro, preferably chloro or bromo.

In addition to anti-secretory activity, certain compounds of formula I possess diuretic activity and they are useful for treatment of conditions requiring diuresis therapy.

This invention provides 4-halo-naphthyridine derivatives which are precursors for the production of the anti-secretory agents and have the formula:

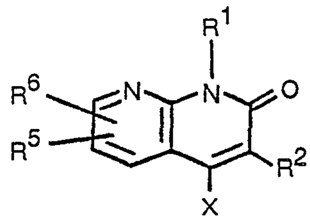

(IV)

in which $R^1$ is hydrogen, alkyl of 1 to 6 carbon atoms, cycloalkyl of 5 to 6 carbon atoms, alken-(3,4,5 or 6)-yl of 3 to 6 carbon atoms, or alkyn-(3,4,5 or 6)-yl of 3 to 6 carbon atoms; $R^2$ is $—CCl_3$, —CN, $—CO_2H$, $—CONR^8R^9$ or $—CO_2R^7$ where $R^7$, $R^8$ and $R^9$ are as defined above; X is chloro, bromo or iodo; and $R^5$ and $R^6$ are, independently hydrogen, halo, alkoxy of 1 to 6 carbon atoms, alkylamino of 1 to 4 carbon atoms, or alkyl of 1 to 4 carbon atoms and salts thereof. The preferred intermediates are those conforming in structure to the preferred group of anti-secretory agents. The compounds of formula IV are converted to the compounds of formula I by processes described in European Patent Application No. 80301157.6 (Publication No. 18735).

This invention also provides processes for preparing the compounds of formula IV.

The compounds of formula IV are prepared by conventional techniques involving displacement of a 4-hydroxy group from the corresponding 4-hydroxy-naphthyridine with such halogenating reagents as phosphorus oxychloride or thionyl chloride to obtain the 4-chloro-naphthyridines and phosphorus oxybromide to obtain the 4-bromo-naphthyridines. The 4-iodo-naphthyridines are prepared by reaction of a 4-chloro-naphthyridine with sodium iodide in an appropriate inert solvent such as acetone.

Alternatively, the 4-chloro, bromo or iodo-naphthyridines may also be prepared directly by nitrosation of the appropriately ring substituted 4-amino-naphthyridines in hydrochloric, hydrobromic or hydroiodic acid, respectively.

Accordingly this invention provides a process for preparing a compound of formula IV which comprises

a) halogenating a compound of formula

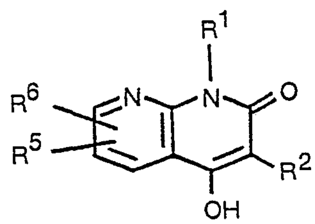

wherein $R^1$, $R^5$ and $R^6$ are as defined above and $R^2$ is $—CONR^8R^9$ or $—CO_2R^7$ wherein $R^7$, $R^8$ and $R^9$ are as defined above, to give a compound of formula IV wherein $R^2$ is CN, $—CONR^8R^9$, $—CO_2R^7$ or $—CCl_3$.

or b) carrying out nitrosation of a compound of formula

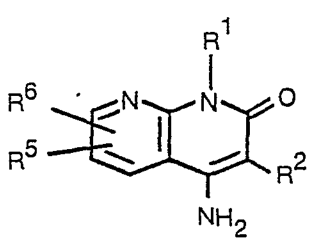

wherein $R^1$, $R^2$, $R^5$, $R^6$ are as herein defined in the presence of an acid of formula HX wherein X is as defined above to give a compound of formula IV,

or c) reacting a compound of formula IV where X is chloro with sodium iodide to give a corresponding compound of formula IV wherein X is iodo.

For example, the 3-trichloromethyl-4-chloro-1,8-naphthyridine precursor is obtained by reaction of the corresponding 3-carboethoxy-4-hydroxy-naphthyridine with $PCl_5$, as shown below:

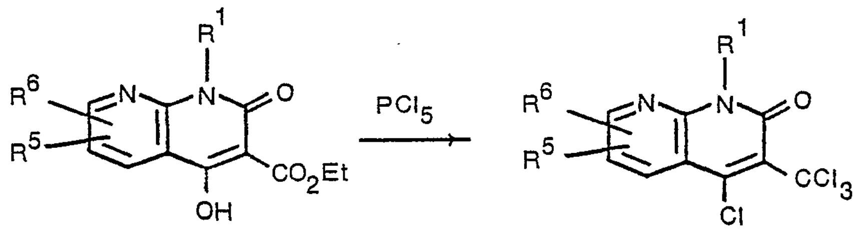


(XIII) (X)

The following Examples 1—5 illustrate the preparation of the compounds of the invention.

Example 1

4-Chloro-1,2-Dihydro-1-Methyl-2-Oxo-1,8-Naphthyridine-3-Carboxylic Acid, Ethyl Ester

To 200 ml. of anhydrous N,N-dimethylformamide was added 1.9. (0.04 mole) of 50% sodium hydride. Then 6.5 g. (0.04 mole) of 3,4-dihydro-1,3-dioxo-1H-pyrido-[2,3-d][1,3] oxazine was added in portions over 5 minutes. After 14.2 g (0.1 mole) of methyl iodide was added, the mixture was stirred at room temperature for 2 hours. The mixture was cooled and water was slowly added. On further dilution of water, a precipitate was formed which was collected, air dried and was recrystallized from ethanol to give 2.6 g. of 1-methyl-1H-4H-pyrido [2,3-d][1,3] oxazine-2,4-dione, m.p. 163—166°C.

| | | | |
|---|---|---|---|
| Analysis for: | $C_8H_6N_2O_3$ | | |
| Calculated: | C, 53.93; | H, 3.40; | N, 15.73 |
| Found: | C, 53.60; | H, 3.52; | N, 15.87 |

To a solution of 0.345 g. (0.15 g. atom) of sodium in 30 ml. of ethanol was added 4.8 g. of diethyl malonate. The mixture was stirred at room temperature for 5 minutes and then was evaporated in a rotary evaporator. The residue was dissolved in 30 ml. of N,N-dimethylformamide and 2.67 g (0.015 mole) of 1-methyl-1H-4H-pyrido [2,3-d][1,3] oxazine-2,4-dione was added. The mixture was heated under reflux for 10 minutes and the thick mixture was dissolved in 100 ml. of water. The solution was acidified with conc. hydrochloric acid and the precipitate which formed was collected, air dried and a small amount of this 1.7 g. was recrystallized from ethanol to give the analytical sample of 4-hydroxy-1,2-dihydro-1-methyl-2-oxo-1,8-naphthyridine-3-carboxylic acid, ethyl ester, m.p. 158—160°C.

| | | | |
|---|---|---|---|
| Analysis for: | $C_{12}H_{12}N_2O_4$ | | |
| Calculated: | C, 58.06; | H, 4.87; | H, 11.29 |
| Found: | C, 57.86; | H, 4.85; | N, 11.18 |

A stirred mixture of 1.7 g of 4-hydroxy-1,2-dihydro-1-methyl-2-oxo-1,8-naphthyridine-3-carboxylic acid ethyl ester in 25 ml. of phosphorus oxychloride was heated under reflux for 2 hours. The phosphorus oxychloride was removed in a rotary evaporator and the residue was poured into 100 ml. of ice water. The precipitate which formed was collected, air dried and a small amount of the 1.9 g. was recrystallized from heptane twice to give the analytical sample of the title compound, m.p. 132—135°C.

| | | | |
|---|---|---|---|
| Analysis for: | $C_{12}H_{11}ClN_2O_3$ | | |
| Calculated: | C, 54.04; | H, 4.16; | N, 10.51 |
| Found: | C, 53.61; | H, 4.16; | N, 10.51 |

The corresponding intermediates in which $R^5$ and $R^6$ are independently alkyl of 1 to 4 carbon atoms are prepared in the same manner, employing the appropriate alkyl substituted precursor. By analogue procedures, the intermediates for amine introduction in 4-position useful in the preparation of the variously substituted compounds of this invention are readily obtained. Likewise, the initial pyrido-oxazine reactant may be alkylated with alkyl iodides of 1 to 6 carbon atoms as well as with alkenyl and alkynyl iodides of 3 to 6 carbon atoms to afford the corresponding 1-substituted precursors.

Example 2

4-Chloro-1-ethyl-7-methyl-3-(trichloromethyl)-1,8-naphthyridine-2-(1H)-one

To a cold solution of 5.4 g. (0.12 mole) of anhydrous ethylamine in 5 ml. of ethanol was added 11.1 g. (0.06 mole) of 2-chloro-6-methylnicotinic acid methyl ester. The mixture was heated in a glass autoclave over a steam bath for 5 hours. The mixture was then evaporated in a rotary evaporator and the residue was added to 100 ml. of water and was basified with conc. ammonium hydroxide. The mixture was then extracted with 100 ml. of chloroform. The chloroform layer was dried over magnesium sulfate, filtered and was evaporated to give 2-ethylamino-6-methyl-nicotinic acid methyl ester as an oil which was used without further purification.

For characterization purposes, a hydrochloride was prepared by dissolving a few ml. of free base in ethyl acetate and adding dropwise a saturated solution of hydrogen chloride in diethyl ether. A few drops of ethanol were added. The solid which formed was removed by filtration and recrystallized from ethyl acetate-ethanol and dried *in vacuo* at 56°C. (m.p. 123—125°C).

| | | | |
|---|---|---|---|
| Analysis for: | $C_{10}H_{15}ClN_2O_2$ | | |
| Calculated: | C, 52.06; | H, 6.12; | N, 12.15 |
| Found: | C, 51.69; | H, 6.44; | N, 12.00 |

To a solution of 3.98 g. (0.02 mole) of 2-ethyl-amino-6-methylnicotinic acid methyl ester in 50 ml. of anhydrous diethyl ether was added 1.5 g. (0.01 mole) of ethyl malonyl chloride. The mixture was stirred at room temperature for 1 hour. The mixture was filtered and the filtrate was evaporated in a rotary evaporator. The residue was added to a solution of 0.23 g. of sodium in 30 ml. of absolute ethanol and was warmed for 5 minutes. The mixture was cooled and the insoluble material was collected and was dissolved in water. Acidification of the water solution with glacial acetic acid afforded a precipitate which was collected, air dried and was recrystallized from heptane to give 1.2 g. of 1-ethyl-1,2-dihydro-4-hydroxy-7-methyl-2-oxo-1,8-naphthyridine-3-carboxylic acid ethyl ester, m.p. 147—151°C.

| | | | |
|---|---|---|---|
| Analysis for: | $C_{14}H_{16}N_2O_4$ | | |
| Calculated: | C, 60.86; | H, 5.84; | N, 10.14 |
| Found: | C, 60.88; | H, 6.00; | N, 9.99 |

To 1 g. of 1-ethyl-1,2-dihydro-4-hydroxy-7-methyl-2-oxo-1,8-naphthyridine-3-carboxylic acid ethyl ester was added 20 g. of phosphorus pentachloride. The reaction became exothermic. After 10 minutes, heat was applied to the reaction and the mixture was kept under reflux for 1 hour. The hot solution was poured onto 100 ml. of ice and the mixture was extracted with 50 ml. of diethyl ether. The ether layer was dried over magnesium sulfate, filtered and was evaporated in a rotary evaporator. The residue was recrystallized from ethyl acetate to afford 0.1 g. of the title compound, m.p. 158—160°C.

| | | | |
|---|---|---|---|
| Analysis for: | $C_{12}H_{10}Cl_4N_2O$ | | |
| Calculated: | C, 42.38; | H, 2.96; | N, 8.24 |
| Found: | C, 42.62; | H, 3.04; | N, 8.26 |

Example 3

4-Chloro-1,7-Dimethyl-1,2-Dihydro-2-Oxo-1,8-Naphthyridine-3-Carboxylic Acid Ethyl Ester

The title compound is produced by the reaction of thionyl chloride and the corresponding 4-hydroxy precursor in a manner enalogous to the procedure of Example 1.

Example 4

4-Chloro-1-Ethyl-1,2-Dihydro-7-Methyl-2-Oxo-1,8-Naphthyridine-3 -Carboxylic Acid Ethyl Ester

The title compound is prepared following the procedure in Example 3 from the corresponding 4-hydroxy compound and thionyl chloride or by nitrosation of the corresponding 4-amino compound with $NaNO_2$ and HCl.

Example 5

4-Chloro-1-Ethyl-1,2-Dihydro-7-Methyl-2-Oxo-1,8-Naphthyridine-3-Carbonitrile

The title compound was prepared by reacting 1-ethyl-1,2-dihydro-4-hydroxy-7-methyl-2-oxo-1,8-naphthyridine-3-carboxylic acid ethyl ester (Example 2) with ethanolic ammonia to form the 3-carboxamide, and that product with phosphorus oxychloride.

The use of the intermediates in preparing compounds of formula I is illustrated by the following Examples:—

Example 6

1-Methyl-1,2-Dihydro-2-Oxo-4-(1-Pyrrolidinyl)-1,8-Naphthyridine-3-Carboxylic Acid Ethyl Ester

A stirred mixture of 2.7 g (0.01 mole) of 4-chloro-1,2-dihydro-1-methyl-2-oxo-1,8-naphthyridine-3-carboxylic acid ethyl ester, 0.7 g (0.01 mole) of pyrrolidine and 1.06 g (0.01 mole) of sodium carbonate in 25 ml of ethanol was heated under reflux for 5 hours. The mixture was filtered and the filtrate was cooled in ice. The precipitate which formed was collected and was dissolved in 20 ml of ethyl acetate. The solution was diluted with petroleum ether to the cloudy point. The precipitate which formed was collected to yield 0.5 g of product, m.p. 118—120°C.

| | | | |
|---|---|---|---|
| Analysis for: | $C_{16}H_{19}N_3O_3$ | | |
| Calculated: | C, 63.77; | H, 6.36; | N, 13.95 |
| Found: | C, 63.46; | H, 6.51; | N, 14.01 |

Example 7

1-Allyl-1,2-Dihydro-7-Methyl-2-Oxo-4-(Pyrrolidinyl)-1,8-Naphthyridine-3-Carboxylic Acid Ethyl Ester

The title compound was prepared by reaction of 1-allyl-4-chloro-1,2-dihydro-7-methyl-2-oxo-1,8-naphthyridine-3-carboxylic acid ethyl ester and pyrrolidine following the procedure of Example 6, m.p. 97—103°C.

Example 8

1-Ethyl-1,2-Dihydro-4-(4-Methyl-1-Piperazinyl)-2-Oxo-1,8-Naphthyridine-3-Carboxylic Acid Ethyl Ester

The title compound was prepared in accordance with the procedure of Example 6, employing 4-chloro-1-ethyl-1,2-dihydro-2-oxo-1,8-naphthyridine-3-carboxylic acid ethyl ester and N-methylpiperazine as the reactants, m.p. 230—233°C.

| | | | |
|---|---|---|---|
| Analysis for: | $C_{18}H_{25}ClN_4O_3$ | | |
| Calculated: | C, 56.76; | H, 6.62; | N, 14.71 |
| Found: | C, 56.49; | H, 6.59; | N, 14.50 |

**Claims for the Contracting States: BE CH DE FR IT LI LU NL SE**

1. A compound of formula

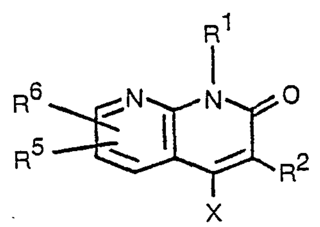

(IV)

in which

$R^1$ is hydrogen, alkyl of 1 to 6 carbon atoms, cycloalkyl of 5 to 6 carbon atoms, alken-(3,4,5 or 6)yl of 3 to 6 carbon atoms or alkyn(3,4,5 or 6)yl of 3 to 6 carbon atoms;

$R^2$ is $—CCl_3$, —CN, $—CO_2H$, $—CO_2R^7$, where $R^7$ is alkyl of 1 to 6 carbon atoms, or $—CONR^8R^9$, where $R^8$ and $R^9$ are independently hydrogen or alkyl of 1 to 6 carbon atoms, X is chloro, bromo or iodo; and

$R^5$ and $R^6$ are independently hydrogen, halo, alkoxy of 1 to 6 carbon atoms, alkylamino of 1 to 4 carbon atoms or alkyl of 1 to 4 carbon atoms, or a salt thereof.

2. A compound of formula (IVa)

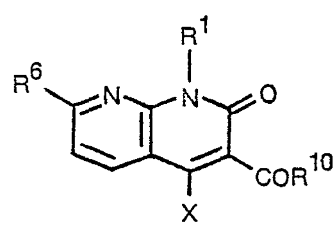

(IVa)

in which $R^1$ is hydrogen, methyl, ethyl, propyl, allyl, propargyl, cyclohexyl or isobutyl; $R^{10}$ is hydroxy or alkoxy of 1 to 6 carbon atoms, and $R^6$ is hydrogen or methyl.

3. 4-Chloro-1,2-dihydro-1-methyl-2-oxo-1,8-naphthyridine-3-carboxylic acid ethyl ester.
4. 4-Chloro-1-ethyl-1,2-dihydro-7-methyl-2-oxo-1,8-naphthyridine-3-carbonitrile.
5. 4-Chloro-1-ethyl-1,2-dihydro-7-methyl-2-oxo-1,8-naphthyridine-3-carboxylic acid, ethyl ester.
6. 4-Chloro-1-ethyl-7-methyl-3-(trichloromethyl)-1,8-naphthyridine-2-(1*H*)-one.
7. 4-Chloro-1-ethyl-1,2-dihydro-2-oxo-1,8-naphthyridine-3-carboxylic acid ethyl ester.
8. 1-Allyl-4-chloro-1,2-dihydro-7-methyl-2-oxo-1,8-naphthyridine-3-carboxylic acid ethyl ester.
9. A process for preparing a compound as claimed in Claim 1 which comprises:

a) halogenating a compound of formula

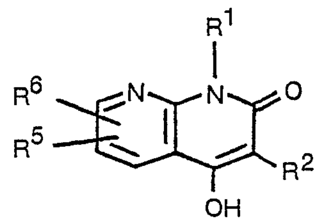

wherein $R^1$, $R^5$ and $R^6$ are as defined in Claim 1 and $R^2$ is $-CONR^8R^9$ or $-CO_2R^7$ wherein $R^7$, $R^8$ and $R^9$ are as defined in Claim 1, to give a compound of formula IV wherein $R^2$ is CN, $-CONR^8R^9$, $-CO_2R^7$ or $-CCl_3$.

or b) carrying out nitrosation of a compound of formula

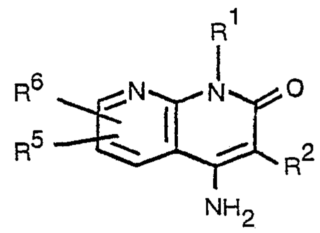

wherein $R^1$, $R^2$, $R^5$, $R^6$ are as defined in Claim 1 in the presence of an acid of formula HX wherein X is as defined in Claim 1 to give a compound of formula IV as defined in Claim 1,

or c) reacting a compound of formula IV where X is chloro with sodium iodide to give a corresponding compound of formula IV wherein X is iodo.

**Claims for the Contracting State: AT**

1. A process for preparing a compound of formula:

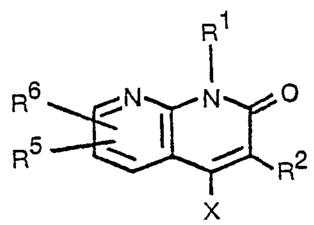

(IV)

in which

$R^1$ is hydrogen, alkyl of 1 to 6 carbon atoms, cycloalkyl of 5 to 6 carbon atoms, alken-(3,4,5 or 6)yl of 3 to 6 carbon atoms or alkyn(3,4,5 or 6)yl of 3 to 6 carbon atoms;

$R^2$ is $-CCl_3$, $-CN$, $-CO_2H$, $-CO_2R^7$ where $R^7$ is alkyl of 1 to 6 carbon atoms, or $-CONR^8R^9$ where $R^8$ and $R^9$ are independently hydrogen or alkyl of 1 to 6 carbon atoms X is chloro, bromo or iodo; and

$R^5$ and $R^6$ are independently hydrogen, halo, alkoxy of 1 to 6 carbon atoms, alkylamino of 1 to 4 carbon atoms or alkyl of 1 to 4 carbon atoms, or a salt thereof,

which comprises

a) halogenating a compound of formula

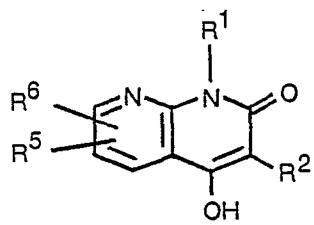

wherein $R^1$, $R^5$ and $R^6$ are as defined above and $R^2$ is $—CONR^8R^9$ or $—CO_2R^7$ wherein $R^7$, $R^8$ and $R^9$ are as defined above, to give a compound of formula IV wherein $R^2$ is —CN, $—CONR^8R^9$, $—CO_2R^7$ or $—CCl_3$,

or b) carrying out nitrosation of a compound of formula

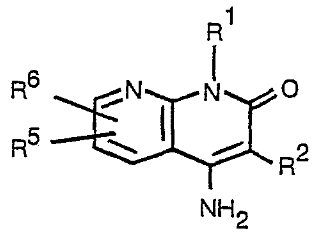

wherein $R^1$, $R^2$, $R^5$, $R^6$ are as defined above in the presence of an acid of formula HX wherein X is as defined in above to give a compound of formula IV as defined above.

or c) reacting a compound of formula IV where X is chloro with sodium iodide to give a corresponding compound of formula IV wherein X is iodo.

2. A process as claimed in Claim 1 in which the product is a compound of formula (IVa)

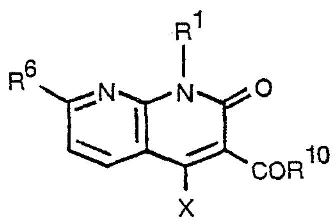

(IVa)

in which $R^1$ is hydrogen, methyl, ethyl, propyl, allyl, propargyl, cyclohexyl or isobutyl;

$R^{10}$ is hydroxy or alkoxy of 1 to 6 carbon atoms, and $R^6$ is hydrogen or methyl.

3. A process as claimed in Claim 1 in which the product is 1-allyl-4-chloro-1,2-dihydro-7-methyl-2-oxo-1,8-naphthyridine-3-carboxylic acid ethyl ester.

4. A process as claimed in Claim 1 in which the product is 4-chloro-1,2-dihydro-1-methyl-2-oxo-1,8-naphthyridine-3-carboxylic acid ethyl ester.

5. A process as claimed in Claim 1 in which the product is 4-chloro-1-ethyl-1,2-dihydro-7-methyl-2-oxo-1,8-naphthyridine-3-carbonitrile.

6. A process as claimed in Claim 1 in which the product is 4-chloro-1-ethyl-1,2-dihydro-7-methyl-2-oxo-1,8-naphthyridine-3-carboxylic acid, ethyl ester.

7. A process as claimed in Claim 1 in which the product is 4-chloro-1-ethyl-7-methyl-3-(trichloromethyl)-1,8-naphthyridine-2-(1*H*)-one.

8. A process as claimed in Claim 1 in which the product is 4-chloro-1-ethyl-1,2-dihydro-2-oxo-1,8-naphthyridine-3-carboxylic acid ethyl ester.

**Patentansprüche für die Vertragsstaaten: BE CH DE FR IT LI LU NL SE**

1. Eine Verbindung der Formel

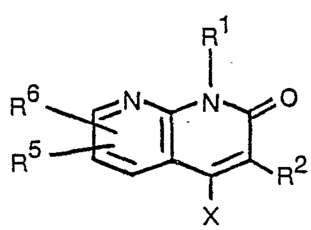

(IV)

worin $R^1$ Wasserstoff, Alkyl mit 1 bis 6 C-Atomen, Cycloalkyl mit 5 bis 6 C-Atomen, Alken-(3,4,5 oder 6)yl mit 3 bis 6 C-Atomen oder Alkin-(3,4,5 oder 6)yl mit 3 bis 6 C-Atomen ist; $R^2$ —$CCl_3$, —CN, —$CO_2H$, —$CO_2R^7$, wobei $R^7$ Alkyl mit 1 bis 6 C-Atomen ist, oder —$CONR^8R^9$ bedeutet, wobei $R^8$ und $R^9$ unabhängig voneinander Wasserstoff oder Alkyl mit 1 bis 6 C-Atomen darstellen; X Chlor, Brom oder Jod ist; und $R^5$ und $R^6$ unabhängig voneinander Wasserstoff, Halogen, Alkoxy mit 1 bis 6 C-Atomen, Alkylamino mit 1 bis 4 C-Atomen oder Alkyl mit 1 bis 4-Catomen bedeuten, oder ein Salz hievon.

2. Eine Verbindung der Formel (IVa)

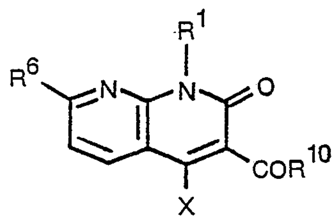

(IVa)

worin $R^1$ Wasserstoff, Methyl, Äthyl, Propyl, Allyl, Propargyl, Cyclohexyl oder Isobutyl; $R^{10}$ Hydroxy oder Alkoxy mit 1 bis 6 C-Atomen und $R^6$ Wasserstoff oder Methyl bedeuten.

3. 4-Chlor-1,2-dihydro-1-methyl-2-oxo-1,8-naphthyridin-3-carbonsäure-äthylester.

4. 4-Chlor-1-äthyl-1,2-dihydro-7-methyl-2-oxo-1,8-naphthyridin-3-carbonitril.

5. 4-Chlor-1-äthyl-1,2-dihydro-7-methyl-2-oxo-1,8-naphthyridin-3-carbonsäure-äthylester.

6. 4-Chlor-1-äthyl-7-methyl-3-(trichlormethy)-1,8-naphthyridin-2-(1H)-on.

7. 4-Chlor-1-äthyl-1,2-dihydro-2-oxo-1,8-naphthyridin-3-carbonsäure-äthylester.

8. 1-Allyl-4-chlor-1,2-dihydro-7-methyl-2-oxo-1,8-naphthyridin-3-carbonsäure-äthylester.

9. Verfahren zum Herstellen einer Verbindung wie in Anspruch 1 beansprucht, welches umfaßt:

a) Halogenieren einer Verbindung der Formel

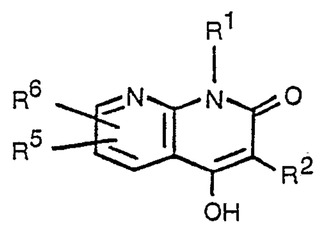

worin $R^1$, $R^5$ und $R^6$ wie in Anspruch 1 definiert sind und $R^2$ die Bedeutung —$CONR^8R^9$ oder —$CO_2R^7$ hat, wobei $R^7$, $R^8$ und $R^9$ wie in Anspruch 1 definiert sind, zu einer Verbindung der Formel (IV), worin $R^2$ —CN, —$CONR^8R^9$, —$CO_2R^7$ oder —$CCl_3$ ist, oder

b) Durchführen der Nitrosierung einer Verbindung der Formel

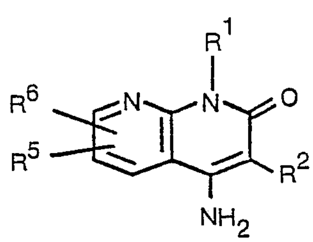

worin $R^1$, $R^2$, $R^5$ und $R^6$ wie in Anspruch 1 definiert sind, in Anwesenheit einer Säure der Formel HX, worin X wie in Anspruch 1 definiert ist, zu einer Verbindung der Formel (IV), wie in Anspruch 1 definiert, oder

c) Umsetzen einer Verbindung der Formel (IV), worin X Chlor bedeutet, mit Natriumjodid zur entsprechenden Verbindung der Formel (IV), worin X Jod bedeutet.

**Patentansprüche für den Vertragsstaat: AT**

1. Verfahren zum Herstellen einer Verbindung der Formel

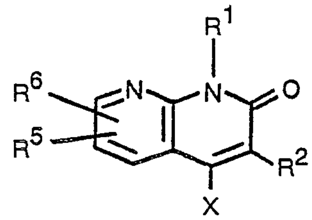

(IV)

worin $R^1$ Wasserstoff, Alkyl mit 1 bis 6 C-Atomen, Cycloalkyl mit 5 bis 6 C-Atomen, Alken-(3,4,5 oder 6)yl mit 3 bis 6 C-Atomen oder Alkin-(3,4,5 oder 6)yl mit 3 bis 6 C-Atomen bedeutet; $R^2$ —$CCl_3$, —CN, —$CO_2H$, —$CO_2R^7$, wobei $R^7$ Alkyl mit 1 bis 6 C-Atomen ist, oder —$CONR^8R^9$ darstellt, wobei $R^8$ und $R^9$ unabhängig voneinander Wasserstoff oder Alkyl mit 1 bis 6 C-Atomen sind; X Chlor, Brom oder Jod bedeutet; und $R^5$ und $R^6$ unabhängig voneinander Wasserstoff, Halogen, Alkoxy mit 1 bis 6 C-Atomen, Alkylamino mit 1 bis 4 C-Atomen oder Alkyl mit 1 bis 4 C-Atomen sind, oder eines Salzes hievon, welches umfaßt:

a) Halogenieren einer Verbindung der Formel

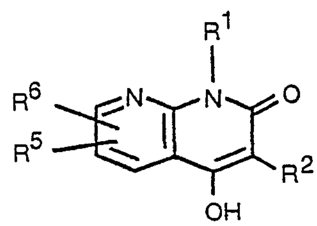

worin $R^1$, $R^5$ und $R^6$ wie oben definiert sind und $R^2$ die Bedeutung —$CONR^8R^9$ oder —$CO_2R^7$ hat, wobei $R^7$, $R^8$ und $R^9$ wie oben definiert sind, zu einer Verbindung der Formel (IV), worin $R^2$ —CN, —$CONR^8R^9$, —$CO_2R^7$ oder —$CCl_3$ ist, oder

b) Durchführen der Nitrosierung einer Verbindung der Formel

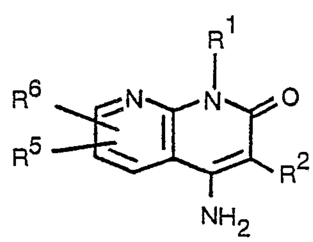

worin $R^1$, $R^2$, $R^5$ und $R^6$ wie oben definiert sind, in Anwesenheit einer Säure der Formel HX, worin X wie oben definiert ist, zu einer Verbindung der Formel (IV), wie oben definiert, oder

c) Umsetzen einer Verbindung der Formel (IV), worin X Chlor bedeutet, mit Natriumjodid zu einer entsprechenden Verbindung der Formel (IV), worin X Jod darstellt.

2. Verfahren wie in Anspruch 1 beansprucht, in dem das Produkt eine Verbindung der Formel (IVa)

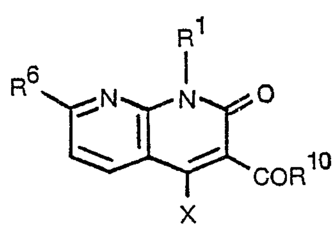

(IVa)

ist, worin $R^1$ Wasserstoff, Methyl, Äthyl, Propyl, Allyl, Propargyl, Cyclohexyl oder Isobutyl, $R^{10}$ Hydroxy oder Alkoxy mit 1 bis 6 C-Atomen und $R^6$ Wasserstoff oder Methyl bedeuten.

3. Verfahren wie in Anspruch 1 beansprucht, worin das Produkt 1-Allyl-4-chlor-1,2-dihydro-7-methyl-2-oxo-1,8-naphthyridin-3-carbonsäure-äthylester ist.

4. Verfahren wie in Anspruch 1 beansprucht, worin das Produkt 4-Chlor-1,2-dihydro-1-methyl-2-oxo-1,8-naphthyridin-3-carbonsäure-äthylester ist.

5. Verfahren wie in Anspruch 1 beansprucht, worin das Produkt 4-Chlor-1-äthyl-1,2-dihydro-7-methyl-2-oxo-1,8-naphthyridin-3-carbonitril ist.

6. Verfahren wie in Anspruch 1 beansprucht, worin das Produkt 4-Chlor-1-äthyl-1,2-dihydro-7-methyl-2-oxo-1,8-naphthyridin-3-carbonsäure-äthylester ist.

7. Verfahren wie in Anspruch 1 beansprucht, worin das Produkt 4-Chlor-1-äthyl-7-methyl-3-(trichlormethyl)-1,8-naphthyridin-2-(1H)-on ist.

8. Verfahren wie in Anspruch 1 beansprucht, worin das Produkt 4-Chlor-1-äthyl-1,2-dihydro-2-oxo-1,8-naphthyridin-3-carbonsäure-äthylester ist.

**Revendications pour les Etats contractants: BE CH DE FR IT LI LU NL SE**

1. Composé de formule

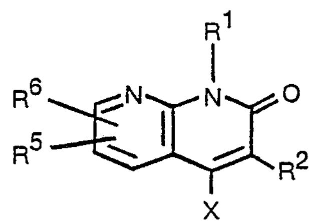

(IV)

dans laquelle

$R^1$ est l'hydrogène, un groupe alkyle ayant 1 à 6 atomes de carbone, un groupe cycloalkyle ayant 5 à 6 atomes de carbone, un groupe alcène-(3,4,5 ou 6)yle ayant 3 à 6 atomes de carbone ou un groupe alcyne-(3,4,5 ou 6)yle ayant 3 à 6 atomes de carbone;

$R^2$ est un groupe $—CCl_3$, —CN, $—CO_2H$, $—CO_2R^7$ où $R^7$ est un radical alkyle ayant 1 à 6 atomes de carbone, ou un groupe $—CONR^8R^9$, dans lequel $R^8$ et $R^9$ représentent, indépendamment, l'hydrogène ou un radical alkyle ayant 1 à 6 atomes de carbone, X est un radical chloro, bromo ou iodo; et

$R^5$ et $R^6$ représentent, indépendamment, l'hydrogène, un radical halogéno, alkoxy ayant 1 à 6 atomes de carbone, alkylamino ayant 1 à 4 atomes de carbone ou alkyle ayant 1 à 4 atomes de carbone, ou sel de ce composé.

2. Composé de formule (IVa)

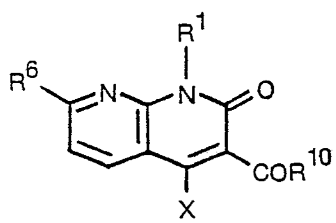

(IVa)

dans laquelle $R^1$ est l'hydrogène, un groupe méthyle, éthyle, propyle, allyle, propargyle, cyclohexyle ou isobutyle;

$R^{10}$ est un groupe hydroxy ou alkoxy ayant 1 à 6 atomes de carbone, et $R^6$ est l'hydrogène ou le groupe méthyle.

3. L'ester éthylique de l'acide 4-chloro 1,2-dihydro-1-méthyl-2-oxo-1,8-naphtyridine-3-carboxylique.

4. Le 4-chloro-1-éthyl-1,2-dihydro-7-méthyl-2-oxo-1,8-naphtyridine-3-carbonitrile.

5. L'ester éthylique de l'acide 4-chloro-1-éthyl-1,2-dihydro-7-méthyl-2-oxo-1,8-naphtyridine-3-carboxylique.

6. La 4-chloro-1-éthyl-7-méthyl-3-(trichloro-methyl)-1,8-naphtyridine-2-(1*H*)-one.

7. L'ester éthylique de l'acide 4-chloro-1-éthyl-1,2-dihydro-2-oxo-1,8-naphtyridine-3-carboxylique.

8. L'ester éthylique de l'acide 1-allyl-4-chloro-1,2-dihydro-7-méthyl-2-oxo-1,8-naphtyridine-3-carboxylique.

9. Procédé de préparation d'un composé suivant la revendication 1, qui consiste:

a) à halogéner un composé de formule

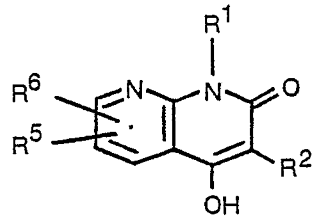

dans laquelle $R^1$, $R^5$ et $R^6$ ont la définition donnée dans la revendication 1 et $R^2$ est un groupe $—CONR^8R^9$ ou $—CO_2R^7$ où $R^7$, $R^8$ et $R^9$ ont les définitions données dans la revendication 1, pour former un composé de formule IV dans laquelle $R^2$ est un groupe CN, $—CONR^8R^9$, $—CO_2R^7$ ou $—CCl_3$, ou

b) à conduire la nitrosation d'un composé de formule

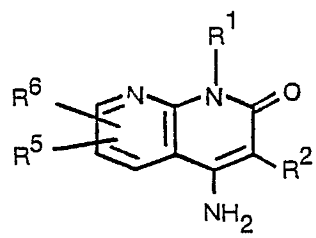

dans laquelle $R^1$, $R^2$, $R^5$, $R^6$ ont les définitions données dans la revendication 1, en présence d'un acide de formule HX dans laquelle X a la définition donnée dans la revendication 1, pour obtenir un composé de formule IV tel que défini dans la revendication 1,

ou

c) à faire réagir un composé de formule IV dans laquelle X est le radical chloro avec l'iodure de sodium pour obtenir un composé correspondant de formule IV dans laquelle X est le radical iodo.

**Revendications pour l'Etat contractant: AT**

1. Procédé de préparation d'un composé de formule:

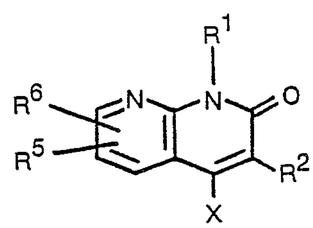

(IV)

dans laquelle

$R^1$ est l'hydrogène, un groupe alkyle ayant 1 à 6 atomes de carbone, un groupe cycloalkyle ayant 5 à 6 atomes de carbone, un groupe alcène-(3,4,5 ou 6)yle ayant 3 à 6 atomes de carbone ou un groupe alcyne-(3,4,5 ou 6)yle ayant 3 à 6 atomes de carbone;

$R^2$ est un groupe $—CCl_3$, $—CN$, $—CO_2H$, $—CO_2R^7$ où $R^7$ est un radical alkyle ayant 1 à 6 atomes de carbone, ou un groupe $—CONR^8R^9$, dans lequel $R^8$ et $R^9$ représentent, indépendamment, l'hydrogène ou un radical alkyle ayant 1 à 6 atomes de carbone, X est un radical chloro, bromo ou iodo; et

$R^5$ et $R^6$ représentent, indépendamment, l'hydrogène, un radical halogéno, alkoxy ayant 1 à 6 atomes de carbone, alkylamino ayant 1 à 4 atomes de carbone ou alkyle ayant 1 à 4 atomes de carbone, ou d'un sel de ce composé, qui consiste

a) à halogéner un composé de formule

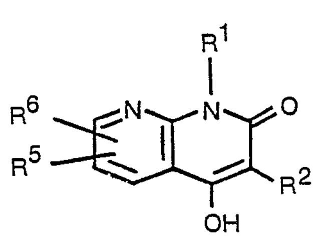

dans laquelle $R^1$, $R^5$ et $R^6$ ont les définitions données ci-dessus et $R^2$ est un groupe $—CONR^8R^9$ ou un groupe $—CO_2R^7$ où $R^7$, $R^8$ et $R^9$ ont les définitions données ci-dessus, pour former un composé de formule IV dans laquelle $R^2$ est un groupe —CN, $—CONR^8R^9$, $—CO_2R^7$ ou $—CCl_3$,

ou

b) à conduire la nitrosation d'un composé de formule

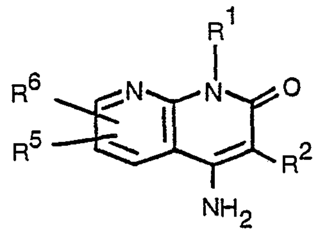

dans laquelle $R^1$, $R^2$, $R^5$, $R^6$ ont les définitions données ci-dessus en présence d'un acide de formule HX dans laquelle X a la définition donnée ci-dessus pour former un composé de formule IV comme défini ci-dessus,

ou

c) à faire réagir un composé de formule IV dans laquelle X est le radical chloro, avec l'iodure de sodium pour former un composé correspondant de formule IV dans laquelle X est le radical iodo.

2. Procédé suivant la revendication 1, dans lequel le produit est un composé de formule (IVa)

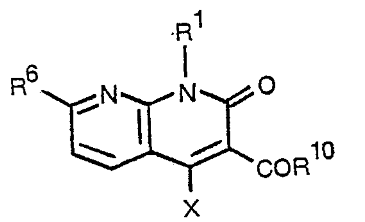

(IVa)

dans laquelle $R^1$ est l'hydrogène, un groupe méthyle, éthyle, propyle, allyle, propargyle, cyclohexyle ou isobutyle;

$R^{10}$ est un groupe hydroxy ou alkoxy ayant 1 à 6 atomes de carbone, et $R^6$ est l'hydrogène ou le groupe méthyle.

3. Procédé suivant la revendication 1, dans lequel le produit est l'ester éthylique de l'acide 1-allyl-4-chloro-1,2-dihydro-7-méthyl-2-oxo-1,8-naphtyridine-3-carboxylique.

4. Procédé suivant la revendication 1, dans lequel le produit est l'ester éthylique de l'acide 4-chloro-1,2-dihydro-1-méthyl-2-oxo-1,8-naphtyridine-3-carboxylique.

5. Procédé suivant la revendication 1, dans lequel le produit est le 4-chloro-1-éthyl-1,2-dihydro-7-méthyl-2-oxo-1,8-naphtyridine-3-carbonitrile.

6. Procédé suivant la revendication 1, dans lequel le produit est l'ester éthylique de l'acide 4-chloro-1-éthyl-1,2-dihydro-7-méthyl-2-oxo-1,8-naphtyridine-3-carboxylique.

7. Procédé suivant la revendication 1, dans lequel le produit est la 4-chloro-1-éthyl-7-méthyl-3-(trichloro-méthyl)-1,8-naphtyridine-2-(1*H*)-one.

8. Procédé suivant la revendication 1, dans lequel le produit est l'ester éthylique de l'acide 4-chloro-1-éthyle-1,2-dihydro-2-oxo-1,8-naphtyridine-3-carboxylique.